# EUROPÄISCHE PATENTANMELDUNG

(11) **EP 4 467 598 A1**
(43) Veröffentlichungstag der Anmeldung: **27.11.2024**
(21) Anmeldenummer: 24175899.4
(22) Anmeldetag: 15.05.2024
(51) Int. Cl.: C08K 5/3447, B60C 1/00, C07D 201/00, C07D 235/04, C07D 235/18

(54) **VERBINDUNG ALS ALTERUNGSSCHUTZMITTEL IN KAUTSCHUKMISCHUNGEN UND FAHRZEUGREIFEN**

(30) Priorität: 23.05.2023 DE 102023204800
(71) Anmelder: Continental Reifen Deutschland GmbH, 30175 Hannover (DE)
(72) Erfinder: Jacob, Andreas, 30175 Hannover (DE); Strohmeier, Julian, 30175 Hannover (DE); Haubold, Thorben Soeren, 30175 Hannover (DE); Schmidt, Philipp, 30175 Hannover (DE); Fleck, Frank, 30175 Hannover (DE); Recker, Carla, 30175 Hannover (DE)
(74) Vertreter: Continental Corporation

(57) **Zusammenfassung**

Die Erfindung betrifft eine Verbindung der Formel (1) 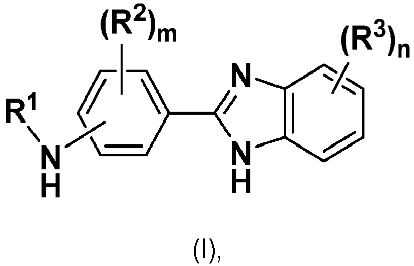 die im vorliegenden Text weiter definiert ist, eine Kautschukmischung enthaltend diese Verbindung, einen Fahrzeugreifen, der die Kautschukmischung in wenigstens einem Bauteil aufweist, ein Verfahren zur Herstellung dieser Verbindung sowie die Verwendung der Verbindung als Alterungsschutzmittel und/oder Antioxidationsmittel.

## Beschreibung

Die Erfindung betrifft eine Verbindung der Formel (I) 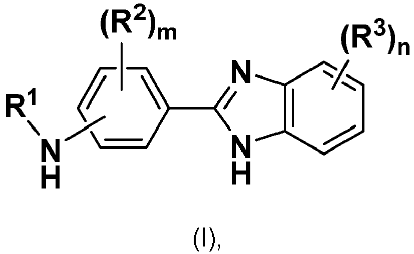 die im vorliegenden Text weiter definiert ist, eine Kautschukmischung enthaltend diese Verbindung, einen Fahrzeugreifen, der die Kautschukmischung in wenigstens einem Bauteil aufweist, ein Verfahren zur Herstellung dieser Verbindung sowie die Verwendung der Verbindung als Alterungsschutz mittel und/oder Antioxidationsmittel.

Es ist bekannt, dass in Fahrzeugreifen und Gummiartikeln, die keine Fahrzeugreifen sind (vorzugsweise technische Gummiartikel), polymere Verbindungen, insbesondere Kautschuke, eingesetzt werden. Naturkautschuk (NR), synthetische Polymere (beispielsweise IR, BR, SSBR, ESBR, etc.) aber auch natürliche sowie synthetische Öle, Fette, Schmier- und Kraftstoffe unterliegen bei längerer Lagerung und häufig in der Zielanwendung, die oft bei höheren Temperaturen abläuft, Oxidationsreaktionen, die sich nachteilig auf die ursprünglich gewünschten Eigenschaften auswirken. Je nach Art der polymeren bzw. langkettigen Verbindung werden diese verkürzt (bis hin zu einer Verflüssigung des Artikels) oder es kommt zu einer nachträglichen, unerwünschten Härtung des Materials bzw. Artikels.

Alterungsschutzmittel tragen daher maßgeblich zu einer verbesserten Langlebigkeit von Fahrzeugreifen bzw. Gummiartikeln und entsprechenden Zusammensetzungen bei.

Typische Alterungsschutzmittel sind aromatische Amine, wie beispielsweise 6PPD (N-(1,3-Dimethylbutyl)-N'-phenyl-p-phenylendiamin), IPPD (N-Isopropyl-N'-phenyl-p-phenylendiamin) oder SPPD (N-(1-phenylethyl)-N'-phenyl-p-phenylendiamin). Diese Verbindungen und Alterungsschutzmittel im Allgemeinen können mit Sauerstoff, Ozon und/oder Radikalen, vorzugsweise Alkyl-, Alkoxy- und Alkylperoxyradikalen, reagieren und diese abfangen und somit die polymeren bzw. langkettigen Verbindungen vor insbesondere unerwünschten Oxidations- und radikalischen Reaktionen schützen. Alterungsschutzmittel, die mit Ozon reagieren bzw. dieses abfangen, werden häufig auch spezifisch "Ozonschutzmittel" oder "Antiozonant" genannt.

Nachteil der oben genannten Verbindungen ist allerdings der Verdacht, dass diese krebserregend und/oder umweltschädlich sein könnten.

Eine weitere Problematik im Zusammenhang mit Alterungsschutzmitteln ist die des unerwünschten Ausblühens (engl. "blooming"). Hierbei diffundieren Moleküle des Alterungsschutzmittels aufgrund ihrer nicht so guten Löslichkeit in der umgebenden Polymer-Matrix des Reifens bzw. Gummiartikels an dessen Oberfläche und bilden dort häufig einen farblich vom restlichen Artikel meist unterscheidbaren Film. Bei Fahrzeugreifen äußert sich dies typischerweise in einer Braunfärbung der ansonsten schwarzen Seitenwand. Neben den ästhetischen Nachteilen sind hiermit Nachteile hinsichtlich der Alterungsschutzwirkung verbunden. Typischerweise werden ausgeblühte Verbindungen nämlich entfernt bzw. durch diesen Vorgang dem Fahrzeugreifen entzogen. Hierbei wird zum einen die Gesamtmenge an Alterungsschutzmittel herabgesetzt und zum anderen bewirkt, dass weitere Moleküle des Alterungsschutzmittels in Richtung Oberfläche nachdiffundieren, sodass die Schutzwirkung immer weiter herabgesetzt wird.

Alterungsschutzmittel in Kautschukzusammensetzungen sind durchaus bekannt. Beispielsweise offenbart US 5,140,055 A Imidazol- und Benzimidazolderivate in Kautschukmischungen.

Der Erfindung liegt die Aufgabe zugrunde, neuartige Verbindungen bereitzustellen, die insbesondere als Alterungsschutzmittel in Fahrzeugreifen und/oder anderen Gummiartikeln, die keine Fahrzeugreifen sind, vorzugsweise technischen Gummiartikeln, verwendet werden können, und zwar mit einem geringeren Gefahrenpotential bei hinreichender Löslichkeit in der jeweiligen Matrix, beispielsweise und insbesondere im Polymer. Hierdurch soll unter Verringerung der Gesundheitsschädlichkeit weiterhin ein Schutz vor Sauerstoff, Radikalen und/oder (vorzugsweise und) Ozon gewährleistet sowie die Tendenz zum Ausblühen (engl. "Blooming") möglichst gering gehalten oder sogar verhindert werden.

Gleichzeitig soll mit der Verbindung eine hinreichende Alterungsschutzwirkung gegenüber bekannten aromatischen Aminen, wie 6PPD, erzielt werden.

Gelöst wird die Aufgabe durch die erfindungsgemäße Verbindung gemäß Formel (I): 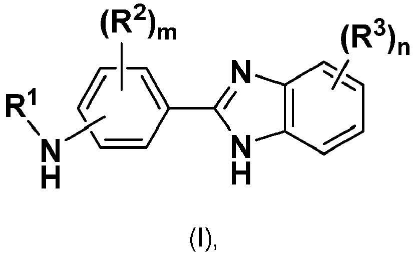 wobei
- R¹ ausgewählt ist aus der Gruppe bestehend aus
   - xi) aromatischen Resten, und
   - xii) aliphatischen C₃- bis C₁₂-Resten,
- R² und R³ gleich oder verschieden sind und unabhängig voneinander ausgewählt sind aus der Gruppe bestehend aus
   aliphatischen C₁- bis C₁₂-Resten, aromatischen Resten, Halogen-Resten, Cyano-Resten, Ester-Resten, Keton-Resten, Ether-Resten und Thioether-Resten,
- m 0 oder 1 oder 2 oder 3 oder 4 ist, und
- n 0 oder 1 oder 2 oder 3 oder 4 ist.

Die erfindungsgemäße Verbindung der Formel (I) ist ein Benzimidazol-Derivat, insbesondere ein Phenyl-Benzimidazol-Derivat, und weist gegenüber bekannten Alterungsschutzmitteln auf Basis von Anilin und deren Abbauprodukten ein geringeres Gefahrenpotential auf. In Fahrzeugreifen und Gummiartikeln, die keine Fahrzeugreifen sind (vorzugsweise technische Gummiartikel), ist dies ein entscheidender Vorteil, da durch Abrieb bzw. andere Abbauprozesse die Gummiinhaltsstoffe häufig freigesetzt werden.

Vorzugsweise ist die erfindungsgemäße Verbindung der Formel (I) ein Ozonschutzmittel, besonders bevorzugt zusätzlich ein Ozonschutzmittel, d.h. zusätzlich zu der Fähigkeit, mit Sauerstoff und/oder Radikalen (vorzugsweise wie oben genannt) reagieren zu können.

Die erfindungsgemäße Verbindung der Formel (I) ist vorzugsweise als Ersatz von 6PPD geeignet, dessen Abbauprodukte insbesondere nachweislich toxisch für den Silberlachs und damit wohl auch für andere aquatische Organismen sind.

Die Indizes m und n beschreiben die Anzahl der entsprechenden Gruppen R² und R³. Sofern m bzw. n gleich 0 (Null) sind, sind die Reste R² bzw. R³ nicht vorhanden und stattdessen durch ein Wasserstoffatom ersetzt. Dies ist vorzugsweise unabhängig voneinander der Fall. Vorzugsweise sind R² und R³ in den erfindungsgemäßen Verbindungen optional.

Dem Fachmann ist ebenfalls klar, dass die Darstellung der Verknüpfungen von (R²)ₘ und NHR¹ an die möglichen Kohlenstoffe im Phenylring und (R³)ₙ am entsprechenden Ring des Benzimidazolgrundgerüstes an alle möglichen Kohlenstoffatome bedeutet. Diese Gruppen sind jeweils an jeder Stelle im jeweils ihnen zugeordneten Ring angeordnet, außer natürlich nicht jeweils zwei oder mehrere gleichzeitig an derselben Position. Im Kontext der vorliegenden Erfindung ist der Rest NHR¹ gleichbedeutend mit N(H)R¹. Es bedeutet, dass es sich um ein sekundäres Amin handelt.

Im Kontext der vorliegenden Erfindung bedeutet die Bezeichnung "C₃- bis C₁₂-Reste" einen Rest umfassend 3 bis 12 Kohlenstoffatome.

Im Kontext der vorliegenden Erfindung gilt grundsätzlich, dass die Erfindung nicht an einen bestimmten Wirkmechanismus oder eine bestimmte Erklärung gebunden sein soll.

Die erfindungsgemäße Verbindung der Formel (I) weist eine hinreichende bis sehr gute Löslichkeit in Kautschukmischungen auf, insbesondere für Fahrzeugreifen und Gummiartikel, die keine Fahrzeugreifen sind (insbesondere technische Gummiartikel). Die Löslichkeit wird vorzugsweise durch geeignete Auswahl der Reste R² und/oder R³ weiter optimiert. Hierdurch wird ein ungewolltes Ausblühen dieser Verbindung geringgehalten oder sogar weitestgehend verhindert, was wiederum vorteilhaft für die Alterungsschutzwirkung ist. Je weniger an Alterungsschutzmittel ausblüht, desto weniger geht davon beabsichtigt oder unbeabsichtigt über die Oberfläche verloren und desto weniger Alterungsschutzmittel diffundiert wiederum nach. Dies hat auch erhebliche Vorteile für die Umwelt.

Von der Erfindung sind sämtliche bevorzugte Ausgestaltungen, die sich unter anderem in den Patentansprüchen widerspiegeln, umfasst. Insbesondere sind von der Erfindung auch Ausgestaltungen umfasst und gelten als offenbart, die sich durch Kombination unterschiedlicher Merkmale ergeben (auch mit jeweils unterschiedlicher Abstufung bei der Bevorzugung dieser Merkmale), sodass auch eine Kombination eines ersten als "bevorzugt" bezeichneten Merkmals mit einem zweiten als z. B. "besonders bevorzugt" bezeichneten Merkmals von der Erfindung umfasst und offenbart sind.

Ferner gelten sämtliche Ausführungen zu den Merkmalen der erfindungsgemäßen Verbindungen auch für das erfindungsgemäße Verfahren zur Herstellung der Verbindungen, die erfindungsgemäße Kautschukmischung enthaltend die Verbindungen sowie die erfindungsgemäße Verwendung.

### Die erfindungsgemäße Verbindung:

Bevorzugt ist eine erfindungsgemäße Verbindung, wobei sie eine Struktur gemäß Formel (II) aufweist: 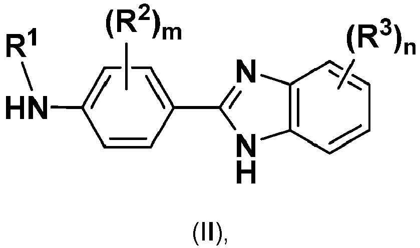 wobei R¹, R², R³, m und n wie vorstehend, vorzugsweise wie im Text als bevorzugt beschrieben, definiert sind.

Das im vorliegenden Text zu der Verbindung gemäß Formel (I) Gesagte gilt besonders bevorzugt *mutatis mutandis* auch für die Verbindung gemäß Formel (II) und umgekehrt. Eine Verbindung der Formel (II) löst die der Erfindung zugrunde liegende Aufgabe besonders gut und zeigt eine hinreichende bis gute Löslichkeit in Kautschukmischungen, insbesondere für Fahrzeugreifen.

Bevorzugt ist eine erfindungsgemäße Verbindung, vorzugsweise wie vorstehend und/oder nachstehend als bevorzugt beschrieben, wobei n und m unterschiedlich oder identisch sind.

Bevorzugt ist eine erfindungsgemäße Verbindung, vorzugsweise wie vorstehend und/oder nachstehend als bevorzugt beschrieben, wobei m und n gleich Null sind.

In der erfindungsgemäßen Verbindung, vorzugsweise wie vorstehend und/oder nachstehend als bevorzugt beschrieben, sind die Reste R² und R³ gleich oder verschieden und unabhängig voneinander ausgewählt aus der Gruppe bestehend aus aliphatischen C₁- bis C₁₂-Resten, aromatischen Resten, Halogen-Resten, Cyano-Resten, Ester-Resten, Keton-Resten, Ether-Resten und Thioether-Resten. Zudem sind R² und R³ voneinander grundsätzlich unabhängig.

Bevorzugt ist eine erfindungsgemäße Verbindung, vorzugsweise wie vorstehend und/oder nachstehend als bevorzugt beschrieben, wobei die aliphatischen C₁- bis C₁₂-Reste in R² und R³ unabhängig voneinander lineare Teilreste, verzweigte Teilreste und/oder cyclische Teilreste umfassen; vorzugsweise linear, verzweigt oder cyclisch sind.

Bevorzugt ist eine erfindungsgemäße Verbindung, vorzugsweise wie vorstehend und/oder nachstehend als bevorzugt beschrieben, wobei die aliphatischen C₁- bis C₁₂-Reste in R² und R³ unabhängig voneinander gesättigt oder ungesättigt sind, vorzugsweise gesättigt.

Bevorzugt ist eine erfindungsgemäße Verbindung, vorzugsweise wie vorstehend und/oder nachstehend als bevorzugt beschrieben, wobei die aliphatischen Reste in R² und R³ aliphatische C₁- bis C₁₀-Reste sind, vorzugsweise aliphatische C₁- bis Cs-Reste, weiter bevorzugt aliphatische C₁- bis C₆-Reste, besonders bevorzugt aliphatische C₁- bis C₄-Reste, ganz besonders bevorzugt aliphatische C₁- bis C₂-Reste, vorzugsweise Methyl und/oder Ethyl.

In einigen Fällen ist eine erfindungsgemäße Verbindung bevorzugt, vorzugsweise wie vorstehend und/oder nachstehend als bevorzugt beschrieben, wobei die aliphatischen C₁- bis C₁₂-Reste in R² und R³ unabhängig voneinander einen oder mehr als einen Halogen-Substituenten umfassen, vorzugsweise ausgewählt aus der Gruppe bestehend aus Fluor, Chlor und Brom.

Bevorzugt ist eine erfindungsgemäße Verbindung, vorzugsweise wie vorstehend und/oder nachstehend als bevorzugt beschrieben, wobei die aromatischen Reste in R² und R³ unabhängig voneinander 5 bis 20 Kohlenstoffatome umfassen, vorzugsweise 6 bis 15 Kohlenstoffatome, weiter bevorzugt 6 bis 12 Kohlenstoffatome, besonders bevorzugt 6 bis 10 Kohlenstoffatome, am bevorzugtesten 6 bis 8 Kohlenstoffatome.

Bevorzugt ist eine erfindungsgemäße Verbindung, vorzugsweise wie vorstehend und/oder nachstehend als bevorzugt beschrieben, wobei die aromatischen Reste in R² und R³ unabhängig voneinander einen oder mehr als einen Halogen-Substituenten umfassen, vorzugsweise ausgewählt aus der Gruppe bestehend aus Fluor, Chlor und Brom.

Bevorzugt ist eine erfindungsgemäße Verbindung, vorzugsweise wie vorstehend und/oder nachstehend als bevorzugt beschrieben, wobei die Halogen-Reste in R² und R³ unabhängig voneinander ausgewählt sind aus der Gruppe bestehend aus Fluor, Chlor und Brom.

Bevorzugt ist eine erfindungsgemäße Verbindung, vorzugsweise wie vorstehend und/oder nachstehend als bevorzugt beschrieben, wobei die Ester-Reste, Keton-Reste, Ether-Reste und Thioether-Reste in R² und R³ unabhängig voneinander 1 bis 20 Kohlenstoffatome umfassen, vorzugsweise 1 bis 16 Kohlenstoffatome, weiter bevorzugt 1 bis 12 Kohlenstoffatome, besonders bevorzugt 1 bis 9 Kohlenstoffatome, weiter besonders bevorzugt 1 bis 7 Kohlenstoffatome, am bevorzugtesten 1 bis 5 Kohlenstoffatome. In einigen Fällen ist es bevorzugt, dass die vorstehenden Obergrenzen an Kohlenstoffatome mit mindestens 2 Kohlenstoffatome als Untergrenze kombiniert sind.

Bevorzugt ist eine erfindungsgemäße Verbindung, vorzugsweise wie vorstehend und/oder nachstehend als bevorzugt beschrieben, wobei R² und R³ unabhängig voneinander maximal 12 Kohlenstoffatome umfassen, vorzugsweise 10 Kohlenstoffatome, besonders bevorzugt 6 Kohlenstoffatome, ganz besonders bevorzugt 4 Kohlenstoffatome. Dies bedeutet beispielsweise, dass R³ maximal 10 Kohlenstoffatme umfasst, während R² maximal 6 Kohlenstoffatome umfasst.

Die genannten Reste R² und R³ sind besonders bevorzugt durch Auswahl geeigneter Ausgangsverbindungen bereits angebunden.

Die Anzahl und Art der Substituenten R² bzw. R³ hat bei entsprechender Auswahl einen entscheidenden Einfluss auf die Löslichkeit der erfindungsgemäßen Verbindung innerhalb der erfindungsgemäßen Kautschukmischung und den erfindungsgemäßen Fahrzeugreifen.

Bevorzugt ist eine erfindungsgemäße Verbindung, wobei m gleich Null ist und n gleich 1 ist, und vorzugsweise R³ ein aliphatischer C₁- bis C₁₂-Rest ist. Das vorstehend zu R³ Gesagte und deren besonders bevorzugte Ausführung gilt vorzugsweise entsprechend. Eine solche bevorzugte Verbindung weist in vielen Fällen eine verbesserte Löslichkeit in der erfindungsgemäßen Kautschukmischung auf und löst damit die zugrunde liegende Aufgabe besonders gut. Insbesondere wird damit das unerwünschte Ausblühen weiter gesenkt oder ganz verhindert.

In der erfindungsgemäßen Verbindung ist der Rest R¹ ausgewählt aus der Gruppe bestehend aus
- xi) aromatischen Resten, und
- xii) aliphatischen C₃- bis C₁₂-Resten.

Bevorzugt ist in einigen Fällen eine erfindungsgemäße Verbindung, vorzugsweise wie vorstehend und/oder nachstehend als bevorzugt beschrieben, wobei die aromatischen Reste in xi) einen Substituenten umfassen ausgewählt aus der Gruppe bestehend aus Alkyl-Resten, Halogen-Resten, Cyano-Resten, Ester-Resten, Keton-Resten, Ether-Resten und Thioether-Resten; besonders bevorzugt Alkyl-Resten, Ester-Resten, Keton-Resten, Ether-Resten und Thioether-Resten. In anderen Fällen und besonders bevorzugt ist es, dass die aromatischen Reste in xi) keinen der oben genannten Substituenten tragen, vorzugsweise gar nicht substituiert sind. Bevorzugt ist der Substituent in meta- oder para-Position. Besonders bevorzugt ist der Substituent nicht in ortho-Position. Dies ist besonders vorteilhaft für die Langlebigkeit des Alterungsschutzmittels.

Bevorzugt ist eine erfindungsgemäße Verbindung, vorzugsweise wie vorstehend und/oder nachstehend als bevorzugt beschrieben, wobei die aromatischen Reste in xi) 5 bis 20 Kohlenstoffatome umfassen, vorzugsweise 6 bis 15 Kohlenstoffatome, weiter bevorzugt 6 bis 10 Kohlenstoffatome, am bevorzugtesten 6 bis 8 Kohlenstoffatome.

Besonders bevorzugt ist eine erfindungsgemäße Verbindung, vorzugsweise wie vorstehend und/oder nachstehend als bevorzugt beschrieben, wobei die aromatischen Reste in xi) Phenyl-Reste (d.h. -C₆H₅), alpha-Methylbenzyl (d.h. - CH₂(CH₃)-C₆H₅), und/oder Benzyl-Reste (d.h. -CH₂-C₆H₅) umfassen, besonders bevorzugt Phenyl-Reste.

Bevorzugt ist eine erfindungsgemäße Verbindung, vorzugsweise wie vorstehend und/oder nachstehend als bevorzugt beschrieben, wobei die aliphatischen C₃- bis C₁₂-Reste in xii) lineare Teilreste, verzweigte Teilreste und/oder cyclische Teilreste umfassen; vorzugsweise linear, verzweigt oder cyclisch sind, am bevorzugtesten verzweigt sind.

Bevorzugt ist eine erfindungsgemäße Verbindung, vorzugsweise wie vorstehend und/oder nachstehend als bevorzugt beschrieben, wobei die aliphatischen C₃- bis C₁₂-Reste in xii) gesättigt oder ungesättigt sind, vorzugsweise gesättigt.

Besonders bevorzugt ist eine erfindungsgemäße Verbindung, vorzugsweise wie vorstehend und/oder nachstehend als bevorzugt beschrieben, wobei in xii) die aliphatischen Reste aliphatische C₃- bis C₁₀-Reste sind, vorzugweise aliphatische C₄- bis Cs-Reste, weiter bevorzugt aliphatische C₅- bis C₇-Reste.

Bevorzugt ist eine erfindungsgemäße Verbindung, vorzugsweise wie vorstehend und/oder nachstehend als bevorzugt beschrieben, wobei R¹ ein tertiäres Kohlenstoffatom umfasst mit dem es an das Stickstoffatom (N) gebunden ist. Das Stickstoffatom (N) ist vorzugsweise ein sekundäres Stickstoff-Atom.

Unter dem Begriff "tertiäres Kohlenstoffatom" ist im Kontext der vorliegenden Erfindung ein Kohlenstoffatom zu verstehen, welches nur ein angebundenes Wasserstoffatom aufweist. Hiermit ergibt sich - gegenüber sekundären und quartären Kohlenstoffatomen - eine besonders gute Schutzwirkung, vorzugsweise in Kautschukmischungen (besonders bevorzugt für Fahrzeugreifen und/oder andere Gummiartikel, die keine Fahrzeugreifen sind (besonders technische Gummiartikel)). Dabei wird insbesondere eine optimierte Reaktivität im Zusammenhang mit den für den Alterungsschutz relevanten Mechanismen erzielt, wobei unerwünschte Nebenreaktionen vermieden werden.

Besonders bevorzugt ist eine erfindungsgemäße Verbindung, wobei R¹ ein verzweigter oder cyclischer Alkyl-Rest ist, vorzugsweise jeweils umfassend 3 bis 12 Kohlenstoffatome, bevorzugt jeweils umfassend 3 bis 8 Kohlenstoffatome, besonders bevorzugt jeweils umfassend 4 bis 7 Kohlenstoffatome. Besonders bevorzugt sind diese gesättigt. Für cyclische Alkyl-Reste gilt vorzugsweise, dass sie mindestens 5 Kohlenstoffatome umfassen, vorzugsweise mindestens 6. Diese Mindestanzahl ist vorzugsweise mit den zuvor genannten Obergrenzen an Kohlenstoffatomen kombiniert.

Sehr besonders bevorzugt ist eine erfindungsgemäße Verbindung, vorzugsweise wie vorstehend und/oder nachstehend als bevorzugt beschrieben, wobei R¹ 1,3-Dimethylbutyl, Phenyl, Benzyl oder Cyclohexyl ist, vorzugsweise 1,3-Dimethylbutyl. Hiermit wird eine besonders gute Schutzwirkung in Kautschukmischungen erzielt, besonders bevorzugt für Fahrzeugreifen und Gummiartikel, die keine Fahrzeugreifen sind (vorzugsweise technische Gummiartikel).

Ganz besonders bevorzugt ist eine erfindungsgemäße Verbindung, wobei sie eine Struktur gemäß Formel (III) aufweist:

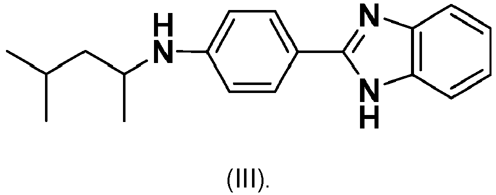

Die Verbindung gemäß Formel (III) weist eine hinreichende Löslichkeit in Polymeren auf, vorzugsweise in Kautschukmischungen, besonders bevorzugt in Kautschukmischungen für Fahrzeugreifen und Gummiartikel, die keine Fahrzeugreifen sind (vorzugsweise technische Gummiartikel). Weiterhin lässt sich die Verbindung gemäß Formel (III) vergleichsweise einfach sowie energie- und kostensparend herstellen und zeigt im Vergleich zu 6PPD eine dramatisch verbesserte Schutzwirkung (siehe Beispiele unten). Die erfindungsgemäße Verbindung gemäß Formel (III) wird nach IPUAC auch als 4-(1*H*-benzo[d]imidazol-2-yl)-*N*-(1,3-Dimethylbutylamino)anilin bezeichnet.

### Kautschukmischung umfassend eine Verbindung gemäß Formel (I):

Die vorliegende Erfindung betrifft zudem eine Kautschukmischung enthaltend die erfindungsgemäße Verbindung wie vorstehend beschrieben (vorzugsweise wie vorstehend und/oder nachfolgend als bevorzugt beschrieben), vorzugsweise umfassend einen oder mehr als einen Dienkautschuk. Die Kautschukmischung umfasst dabei eine oder mehr als eine erfindungsgemäße Verbindung, wobei vorzugsweise mindestens ein Kautschuk ein Dienkautschuk ist.

Das vorstehend Gesagte zu der erfindungsgemäßen Verbindung, insbesondere wie vorstehend als bevorzugt beschrieben, gilt vorzugsweise *mutatis mutandis* auch für die erfindungsgemäße Kautschukmischung.

Die erfindungsgemäße Kautschukmischung enthält somit wenigstens einen Kautschuk. Bei der erfindungsgemäßen Kautschukmischung handelt es sich prinzipiell um jede beliebige Kautschukmischung in der die erfindungsgemäßen Verbindungen als Alterungsschutzmittel und/oder Ozonschutzmittel bei geringerer Toxizität wirken. Die erfindungsgemäße Kautschukmischung umfasst einen oder mehr als einen Kautschuk.

Bevorzugt ist eine erfindungsgemäße Kautschukmischung, vorzugsweise wie vorstehend und/oder nachstehend als bevorzugt beschrieben, umfassend eine oder mehr als eine erfindungsgemäße Verbindung (vorzugsweise wie vorstehend als bevorzugt beschrieben), wobei diese die einzigen Benzimidazolverbindungen in der Kautschukmischung sind, besonders bevorzugt sind die Verbindung der Formel (II) oder (III) die einzigen Benzimidazolverbindungen in der Kautschukmischung.

Bevorzugt ist eine erfindungsgemäße Kautschukmischung, vorzugsweise wie vorstehend und/oder nachstehend als bevorzugt beschrieben, umfassend eine Gesamtmenge der erfindungsgemäßen Verbindung von 0,1 bis 10 phr, bevorzugt von 0,3 bis 8 phr, weiter bevorzugt von 0,6 bis 6 phr, besonders bevorzugt von 0,8 bis 4,5 phr, ganz besonders bevorzugt von 1 bis 3 phr.

Die im Kontext der vorliegenden Erfindung verwendete Angabe phr (parts per hundred parts of rubber by weight) ist die in der Kautschukindustrie übliche Mengenangabe für Mischungsrezepturen. Die Dosierung der Gewichtsteile wird auf 100 Gewichtsteile der gesamten Masse aller in der Mischung vorhandenen hochmolekularen (M_{w} größer als 20.000 g/mol) Kautschuke bezogen.

Die erfindungsgemäße Kautschukmischung, vorzugsweise wie vorstehend und/oder nachstehend als bevorzugt beschrieben, umfasst vorzugsweise einen oder mehr als einen Dienkautschuk. Als Dienkautschuke werden typischerweise Kautschuke bezeichnet, die durch Polymerisation oder Copolymerisation von Dienen und/oder Cycloalkenen entstehen und somit entweder in der Hauptkette oder in den Seitengruppen C=C-Doppelbindungen aufweisen.

Bevorzugt ist eine erfindungsgemäße Kautschukmischung, vorzugsweise wie vorstehend und/oder nachstehend als bevorzugt beschrieben, wobei der Dienkautschuk ausgewählt ist aus der Gruppe bestehend aus natürlichem Polyisopren (NR), synthetischem Polyisopren (IR), epoxidiertem Polyisopren (ENR), Butadien-Kautschuk (BR), Butadien-Isopren-Kautschuk, lösungspolymerisiertem Styrol-Butadien-Kautschuk (SSBR), emulsionspolymerisiertem Styrol-Butadien-Kautschuk (ESBR), Styrol-Isopren-Kautschuk, Flüssigkautschuken mit einem Molekulargewicht M_{w} von größer als 20000 g/mol, Butylkautschuk (IIR), Halobutyl-Kautschuk (XIIR), Polynorbornen, Isopren-Isobutylen-Copolymer, Ethylen-Propylen-Dien-Kautschuk, Nitril-Kautschuk, Chloropren-Kautschuk, Acrylat-Kautschuk, Fluor-Kautschuk, SilikonKautschuk, Polysulfid-Kautschuk, Epichlorhydrin-Kautschuk, Styrol-Isopren-Butadien-Terpolymer, hydriertem Acrylnitrilbutadien-Kautschuk und hydriertem Styrol-Butadien-Kautschuk.

Im Kontext der vorliegenden Erfindung ist unter dem Begriff "Naturkautschuk" und "natürlicher Kautschuk", beide Begriffe haben einen identischen Bedeutungsumfang, natürlich vorkommender Kautschuk zu verstehen, der von Hevea Gummibäumen und/oder "Nicht-Hevea" Quellen gewonnen wird.

Bevorzugte Nicht-Hevea Quellen sind Guayule Sträucher und Löwenzahn. Ein besonders bevorzugter Löwenzahn ist TKS (Taraxacum kok-saghyz; Russischer Löwenzahn).

Besonders bevorzugt ist eine erfindungsgemäße Kautschukmischung, vorzugsweise wie vorstehend und/oder nachstehend als bevorzugt beschrieben, wobei der Dienkautschuk ausgewählt ist aus der Gruppe bestehend aus natürlichem Polyisopren (NR), synthetischem Polyisopren (IR), Butadien-Kautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutyl-Kautschuk (XIIR).

Ganz besonders bevorzugt ist eine erfindungsgemäße Kautschukmischung, vorzugsweise wie vorstehend und/oder nachstehend als bevorzugt beschrieben, wobei der Dienkautschuk ausgewählt ist aus der Gruppe bestehend aus natürlichem Polyisopren (NR), synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Nitril-Kautschuk, hydrierter Acrylnitrilbutadien-Kautschuk, Chloropren-Kautschuk, Butylkautschuk (IIR), Halobutylkautschuk (XIIR) und/oder Ethylen-Propylen-Dien-Kautschuk kommen vorzugsweise bei der Herstellung von Gummiartikeln, die keine Fahrzeugreifen sind (aber sonst typischerweise als technische Gummiartikel gelten) zum Einsatz, vorzugsweise für Gurte, Riemen Schläuche, Bänder und/oder Schuhsohlen. Dabei finden die dem Fachmann für diese Kautschuke bekannten - im Hinblick auf Füllstoffe, Weichmacher, Vulkanisationssysteme und Zuschlagstoffe besonderen - Mischungszusammensetzungen bevorzugte Anwendung.

Vorzugsweise umfasst Polyisopren cis-1,4-Polyisopren und/oder 3,4-Polyisopren. Dies gilt vorzugsweise unabhängig davon, ob es sich um natürliches oder synthetisches Polyisopren handelt. Bevorzugt ist *cis*-1,4-Polyisopren mit besonders bevorzugt einem cis-1,4 Anteil größer 90 Gew.-%. Solch ein Polyisopren wird vorzugsweise durch stereospezifische Polymerisation in Lösung mit Ziegler-Natta-Katalysatoren oder unter Verwendung von fein verteiltem Lithiumalkylen erhalten. Weiterhin handelt es sich auch bei Naturkautschuk (NR) um ein solches *cis*-1,4 Polyisopren. Besonders bevorzugt ist daher Naturkautschuk (NR) umfassend cis-1,4-Polyisopren, vorzugsweise mit einem cis-1,4-Anteil größer oder gleich 99 Gew.-%.

In einigen Fällen ist eine erfindungsgemäße Kautschukmischung bevorzugt, vorzugsweise wie vorstehend und/oder nachstehend als bevorzugt beschrieben, umfassend ein oder mehr als ein natürliches Polyisopren (NR) und ein oder mehr als ein synthetisches Polyisopren (IR). Es liegt somit vorzugsweise ein Gemisch aus Polyisopren vor.

In einigen Fällen ist eine erfindungsgemäße Kautschukmischung besonders bevorzugt, vorzugsweise wie vorstehend und/oder nachstehend als bevorzugt beschrieben, umfassend ein oder mehr als ein natürliches Polyisopren (NR), vorzugsweise in einer Gesamtmenge von 56 bis 130 phr, besonders bevorzugt von 80 bis 120 phr, ganz besonders bevorzugt von 90 bis 110 phr, weiter bevorzugt von 95 bis 100 phr. Eine derartige Kautschukmischung zeigt insbesondere optimierte Reißeigenschaften und Abriebeigenschaften bei gleichzeitig guter Verarbeitbarkeit und Reversionsstabilität. Eine weitere bevorzugte Gesamtmenge liegt im Bereich von 56 bis 100 phr, vorzugsweise von 80 bis 100 phr, weiter bevorzugt von 90 bis 100 phr.

In einigen Fällen ist eine erfindungsgemäße Kautschukmischung bevorzugt, vorzugsweise wie vorstehend und/oder nachstehend als bevorzugt beschrieben, umfassend ein oder mehr als ein natürliches Polyisopren (NR), wobei dessen Gesamtmenge signifikant weniger als 100 phr beträgt. In diesen Fällen umfasst die Kautschukmischung zusätzlich einen oder mehr als einen von natürlichem Polyisopren verschiedenen Kautschuk, vorzugsweise ein weiteren Dienkautschuk, besonders bevorzugt ausgewählt aus der Gruppe bestehend aus synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR). Besonders bevorzugt ist in diesen Fällen eine erfindungsgemäße Kautschukmischung umfassend ein oder mehr als ein natürliches Polyisopren (NR) in einer Gesamtmenge von 5 bis 55 phr, vorzugsweise von 5 bis 35 phr, weiter bevorzugt von 5 bis 25 phr, ganz besonders bevorzugt von 5 bis 20 phr. Eine derartige Kautschukmischung zeigt insbesondere eine gute Verarbeitbarkeit und Reversionsstabilität sowie optimierte Reißeigenschaften und ein optimales Rollwiderstandsverhalten.

In einigen Fällen ist eine erfindungsgemäße Kautschukmischung bevorzugt, vorzugsweise wie vorstehend und/oder nachstehend als bevorzugt beschrieben, umfassend mindestens einen Butadien-Kautschuk (BR, Polybutadien). Bevorzugt ist ein Butadien-Kautschuk umfassend einen high-cis- und/oder einen low-*cis*-Typ, wobei Polybutadien mit einem cis-Anteil größer oder gleich 90 Gew.-% als high-cis-Typ und Polybutadien mit einem cis-Anteil kleiner 90 Gew.-% als low-cis-Typ bezeichnet wird. Ein besonders bevorzugter low-cis-Typ umfasst Li-BR (Lithiumkatalysierter Butadien-Kautschuk), vorzugsweise mit einem cis-Anteil von 20 bis 50 Gew.-%. Besonders bevorzugt ist ein high-cis-Typ, da damit besonders gute Eigenschaften, insbesondere eine niedrige Hysterese der Kautschukmischung, erzielt werden.

In einigen Fällen ist eine erfindungsgemäße Kautschukmischung bevorzugt, vorzugsweise wie vorstehend und/oder nachstehend als bevorzugt beschrieben, wobei der Butadien-Kautschuk mittels einer oder mehr als einer funktionalen Gruppe endgruppenfunktionalisiert und/oder entlang der Polymerketten funktionalisiert ist. Vorzugsweise ist die eine oder mehr als eine funktionale Gruppe unabhängig voneinander ausgewählt aus der Gruppe bestehend aus Hydroxy-Gruppen, Ethoxy-Gruppen, Epoxy-Gruppen, Siloxan-Gruppen, AminoGruppen, Aminosiloxan-Gruppen, Carboxy-Gruppen, Phthalocyanin-Gruppen und Silan-Sulfid-Gruppen. Die Auswahl der funktionalen Gruppe ist jedoch vorzugsweise nicht auf die vorstehenden Gruppen beschränkt. In einigen Fällen umfasst die funktionale Gruppe vorzugsweise ein oder mehrere Metalle.

Bevorzugt ist eine erfindungsgemäße Kautschukmischung, vorzugsweise wie vorstehend und/oder nachstehend als bevorzugt beschrieben, umfassend den einen oder mehr als einen Butadien-Kautschuk in einer Gesamtmenge von 10 bis 80 phr, vorzugsweise von 12 bis 50 phr, besonders bevorzugt von 15 bis 40 phr. Hiermit werden besonders gute Reiß- und Abriebeigenschaften der erfindungsgemäßen Kautschukmischung und ein optimales Bremsverhalten erzielt.

In einigen Fällen ist eine erfindungsgemäße Kautschukmischung bevorzugt, vorzugsweise wie vorstehend und/oder nachstehend als bevorzugt beschrieben, umfassend mindestens ein Styrol-Butadien-Kautschuk (Styrol-Butadien-Copolymer). Bevorzugt ist ein Styrol-Butadien-Kautschuk umfassend einen oder mehr als einen lösungspolymerisierten Styrol-Butadien-Kautschuk (SSBR) und/oder einen oder mehr als einen emulsionspolymerisierten Styrol-Butadien-Kautschuk (ESBR). Im Kontext der vorliegenden Erfindung werden die Begriffe "Styrol-Butadien-Kautschuk" und "Styrol-Butadien-Copolymer" synonym verwendet. In einigen Fällen ist eine erfindungsgemäße Kautschukmischung bevorzugt, wobei der Styrol-Butadien-Kautschuk mittels einer oder mehr als einer funktionalen Gruppe endgruppenfunktionalisiert und/oder entlang der Polymerketten funktionalisiert ist. Zu bevorzugten funktionalen Gruppen gilt das vorstehend zum Butadien-Kautschuk Gesagte entsprechend.

Bevorzugt ist eine erfindungsgemäße Kautschukmischung, vorzugsweise wie vorstehend und/oder nachstehend als bevorzugt beschrieben, umfassend den mindestens einen Styrol-Butadien-Kautschuk (SSBR) in einer Gesamtmenge von 10 bis 80 phr, bevorzugt von 30 bis 75 phr, besonders bevorzugt von 50 bis 70 phr. Besonders bevorzugt ist eine erfindungsgemäße Kautschukmischung umfassend einen oder mehr als einen lösungspolymerisierter Styrol-Butadien-Kautschuk (SSBR) welcher zudem in einer Gesamtmenge von 10 bis 80 phr vorliegt, bevorzugt von 30 bis 75 phr, besonders bevorzugt von 50 bis 70 phr. Hiermit werden besonders gute Rollwiderstandseigenschaften der erfindungsgemäßen Kautschukmischung erzielt. Besonders bevorzugt wird der lösungspolymerisierte Styrol-Butadien-Kautschuk (SSBR) in Kombination mit einem oder mehr als einem weiteren Kautschuk eingesetzt. Dadurch wird typischerweise ein verbessertes und ausbalancierteres Eigenschaftsprofil erzielt.

Neben einer oder mehr als einer erfindungsgemäßen Verbindung und mindestens einem Kautschuk umfasst die erfindungsgemäße Kautschukmischung vorzugsweise zusätzlich Inhaltsstoffe, vorzugsweise mindestens einen Füllstoff.

Bevorzugt ist eine erfindungsgemäße Kautschukmischung, vorzugsweise wie vorstehend und/oder nachstehend als bevorzugt beschrieben, zusätzlich umfassend einen oder mehr als einen Füllstoff, vorzugsweise in einer Gesamtmenge von 30 bis 500 phr, bevorzugt von 50 bis 400 phr, weiter bevorzugt von 80 bis 300 phr. Ein besonders bevorzugter Füllstoff ist ein verstärkender Füllstoff, besonders bevorzugt ausgewählt aus der Gruppe bestehend aus Rußen und Siliciumdioxiden.

Als Ruß kommen grundsätzlich alle dem Fachmann bekannten Rußtypen in Frage. Bevorzugt ist der Ruß ausgewählt aus der Gruppe bestehend aus Industrieruß und Pyrolyse-Ruß, wobei ein Industrieruß bevorzugt ist.

Bevorzugt hat der Ruß eine Jodzahl, gemäß ASTM D 1510 (die auch als Jodadsorptionszahl bezeichnet wird), in einem Bereich von 30 bis 250 g/kg, bevorzugt von 35 bis 215 g/kg, besonders bevorzugt von 40 bis 180 g/kg, und ganz besonders bevorzugt von 45 bis 140 g/kg, und/oder eine DBP-Zahl, gemäß ASTM D 2414, in einem Bereich von 30 bis 200 ml/100 g, bevorzugt von 70 bis 170 ml/100 g, besonders bevorzugt von 90 bis 140 ml/100 g. Die DBP-Zahl gemäß ASTM D 2414 bestimmt das spezifische Absorptionsvolumen eines Rußes oder eines hellen Füllstoffes mittels Dibutylphthalat. Die Verwendung eines solchen Rußes in der erfindungsgemäßen Kautschukmischung, insbesondere für Fahrzeugreifen, gewährleistet üblicherweise einen bestmöglichen Kompromiss aus Abriebwiderstand und Wärmeaufbau, der wiederum den ökologisch relevanten Rollwiderstand beeinflusst. Besonders bevorzugt ist ein Ruß mit einer Jodzahl im Bereich von 80 bis 110 g/kg und einer DBP-Zahl im Bereich von 100 bis 130 ml/100 g. Ganz besonders bevorzugt ist ein Ruß des Typs N339.

In manchen Fällen ist eine erfindungsgemäße Kautschukmischung bevorzugt, vorzugsweise wie vorstehend und/oder nachstehend als bevorzugt beschrieben, wobei der Ruß in der Kautschukmischung in einer Gesamtmenge im Bereich von 0,1 bis 60 phr vorliegt, bevorzugt von 3 bis 40 phr, besonders bevorzugt von 4 bis 30 phr, ganz besonders bevorzugt von 5 bis 15 phr. In diesen Mengen ist der Ruß typischerweise als Ergänzungsfüllstoff in Kombination mit einem Hauptfüllstoff, vorzugsweise einem oder mehr als einem Siliciumdioxid, besonders bevorzugt Kieselsäure, enthalten.

In anderen Fällen ist eine erfindungsgemäße Kautschukmischung bevorzugt, vorzugsweise wie vorstehend und/oder nachstehend als bevorzugt beschrieben, wobei der Ruß in der Kautschukmischung in einer Gesamtmenge im Bereich von 30 bis 300 phr vorliegt, bevorzugt von 35 bis 200 phr, besonders bevorzugt von 40 bis 100 phr. In diesen Mengen ist Ruß insbesondere als alleiniger oder als Hauptfüllstoff (mehr als 50 Gew.-% bezogen auf die Gesamtfüllstoffmenge; vorzugsweise in Kombination mit vergleichsweise geringen Mengen an Kieselsäure) enthalten.

Vorzugsweise ist das Siliciumdioxid amorphes Siliciumdioxid und/oder pyrogenes Siliciumdioxid. Ein besonders bevorzugtes amorphes Siliciumdioxid umfasst gefällte Kieselsäure, die auch als gefälltes Siliciumdioxid bezeichnet wird. Im Kontext der vorliegenden Erfindung sind die Begriffe "Kieselsäure" und "Silika" bedeutungsgleich und daher synonym.

Besonders bevorzugt ist amorphes Siliciumdioxid, vorzugsweise gefällte Kieselsäure, mit einer Stickstoff-Oberfläche (BET-Oberfläche) (gemäß DIN ISO 9277 und DIN 66132) im Bereich von 35 bis 400 m²/g, bevorzugt von 50 bis 350 m²/g, besonders bevorzugt von 85 bis 320 m²/g, ganz besonders bevorzugt von 120 bis 235 m²/g, und/oder (vorzugsweise und) mit einer CTAB-Oberfläche (gemäß ASTM D 3765) im Bereich von 30 bis 400 m²/g, bevorzugt von 50 bis 330 m²/g, besonders bevorzugt von 80 bis 300 m²/g, ganz besonders bevorzugt von 115 bis 200 m²/g. Derartiges Siliciumdioxid führt z. B. in Kautschukmischungen für Reifenlaufstreifen zu besonders guten physikalischen Eigenschaften der Vulkanisate. Außerdem können sich dabei Vorteile in der Mischungsverarbeitung durch eine Verringerung der Mischzeit bei gleichbleibenden Produkteigenschaften ergeben, die zu einer verbesserten Produktivität führen. Besonders bevorzugt ist eine Kieselsäure des Typs Ultrasil^{®} VN3 (Handelsname) der Firma Evonik, als auch hoch-dispergierbare Kieselsäuren (sogenannte HD-Kieselsäuren). Bevorzugte hoch-dispergierbare Kieselsäuren umfassen Zeosil^{®} 1165 MP der Firma Solvay.

In manchen Fällen ist eine erfindungsgemäße Kautschukmischung bevorzugt, vorzugsweise wie vorstehend und/oder nachstehend als bevorzugt beschrieben, umfassend mindestens eine Kieselsäure, bevorzugt in einer Gesamtmenge im Bereich von 30 bis 500 phr, weiter bevorzugt von 50 bis 400 phr, besonders bevorzugt von 80 bis 300 phr. In diesen Mengen ist Kieselsäure insbesondere als alleiniger oder als Hauptfüllstoff (mehr als 50 Gew.-% bezogen auf die Gesamtfüllstoffmenge; vorzugsweise in Kombination mit vergleichsweise geringen Mengen an Ruß) enthalten.

In manchen Fällen ist eine erfindungsgemäße Kautschukmischung besonders bevorzugt, vorzugsweise wie vorstehend und/oder nachstehend als bevorzugt beschrieben, umfassend mindestens eine Kieselsäure, bevorzugt in einer Gesamtmenge im Bereich von 5 bis 100 phr, weiter bevorzugt von 7 bis 80 phr, besonders bevorzugt von 10 bis 60 phr. In diesen Mengen ist Kieselsäure typischerweise als Ergänzungsfüllstoff in Kombination mit einem Hauptfüllstoff, vorzugsweise einem Ruß, enthalten.

Besonders bevorzugt ist eine erfindungsgemäße Kautschukmischung, vorzugsweise wie vorstehend und/oder nachstehend als bevorzugt beschrieben, umfassend mindestens eine Kieselsäure und mindestens einen Ruß, wobei die Gesamtmenge an Kieselsäure im Bereich von 50 bis 300 phr, bevorzugt von 80 bis 200 phr, liegt und die Gesamtmenge an Ruß im Bereich von 5 bis 60 phr, bevorzugt von 7 bis 40 phr.

Vorzugsweise umfasst die erfindungsgemäße Kautschukmischung, vorzugsweise wie vorstehend und/oder nachstehend als bevorzugt beschrieben, zusätzliche Füllstoffe, d.h. die keine Ruße, keine Siliciumdioxide bzw. keine Kieselsäuren sind bzw. umfassen. Diese zusätzlichen Füllstoffe umfassen vorzugsweise ebenfalls verstärkende Füllstoffe und/oder nicht-verstärkende Füllstoffe.

Bevorzugte nicht-verstärkende Füllstoffe umfassen Alumosilicate, Kaolin, Kreide, Stärke, Magnesiumoxid, Titandioxid, Kautschukgele und/oder Fasern. Bevorzugte Fasern umfassen Aramidfasern, Glasfasern, Carbonfasern und/oder Cellulosefasern.

Bevorzugte ebenfalls verstärkende Füllstoffe umfassen Kohlenstoffnanoröhrchen, Grafit, Graphene und/oder sogenannte "carbon-silica dual-phase filler". Die Kohlenstoffnanoröhrchen umfassen vorzugsweise unmodifizierte und/oder mit funktionellen Gruppen modifizierte Kohlenstoffnanoröhrchen, wobei bevorzugte funktionelle Gruppen ausgewählt sind aus der Gruppe bestehend aus Hydroxy-, Carboxy und Carbonyl-Gruppen.

Im Kontext der vorliegenden Erfindung gehört Zinkoxid nicht zu den Füllstoffen im Sinne der vorliegenden Erfindung.

Bevorzugt ist eine erfindungsgemäße Kautschukmischung, vorzugsweise wie vorstehend und/oder nachstehend als bevorzugt beschrieben, umfassend einen oder mehr als einen weiteren Zusatzstoff. Hierbei handelt es sich typischerweise um übliche Zusatzstoffe in den typischen Mengen, die bei der Herstellung der Kautschukmischung vorzugsweise in wenigstens eine Grundmischstufe zugegeben werden.

Vorzugsweise umfasst der eine oder mehr als eine weitere Zusatzstoff:
a) Alterungsschutzmittel, die aus dem Stand der Technik bekannt sind und die keine erfindungsgemäße Verbindung der Formel (I), bzw. sonstige erfindungsgemäße Verbindungen sind; vorzugsweise umfassend para-Phenylendiamine und/oder Dihydrochinoline; besonders bevorzugt ausgewählt aus der Gruppe bestehend aus N-Phenyl-N'-(1,3-dimethylbutyl)-p-phenylendiamin (6PPD), N,N`-Diphenyl-p-phenylendiamin (DPPD), N-(1-phenylethyl)-N'-phenyl-p-phenylendiamin (SPPD), N,N`-Ditolyl-p-phenylendiamin (DTPD), N-(1,4-dimethylpentyl)-N'-phenyl-p-phenylendiamin (7PPD), N-Isopropyl-N'-phenyl-p-phenylendiamin (IPPD), und
   2,2,4-Trimethyl-1,2-dihydrochinolin (TMQ);
b) einen Aktivator; vorzugsweise umfassend Zinkverbindungen (inklusive Zinkkomplexe) und/oder Fettsäuren; besonders bevorzugt umfassend Zinkoxid, Stearinsäure und/oder Zinkethylhexanoat;
c) Agenzien für die Anbindung von Füllstoffen (insbesondere für Ruß und Kieselsäure); vorzugsweise umfassend S-(3-Aminopropyl)-Thioschwefelsäure, deren Metallsalze (besonders für die Anbindung an Ruß) und/oder Silan-Kupplungsagenzien (besonders für die Anbindung an Siliciumdioxid, insbesondere an Kieselsäure);
d) Ozonschutzwachse;
e) Harze; vorzugsweise Klebharze;
f) Mastikationshilfsmittel; vorzugsweise umfassend 2,2'-Dibenzamidodiphenyldisulfid (DBD);
g) Prozesshilfsmittel; vorzugsweise umfassend Fettsäureester und/oder Metallseifen, wobei bevorzugte Metallseifen Zinkseifen und/oder Calciumseifen umfassen; und/oder
h) Weichmacher; vorzugsweise umfassend Öle (vorzugsweise umfassend aromatische, naphthenische und/oder paraffinische Mineralöle), Harze und/oder Flüssig-Polymere; besonders bevorzugt umfassend MES (mild extraction solvate), DAE (Destillated Aromatic Extracts), RAE (Residual Aromatic Extract), TDAE (treated distillate aromatic extract), Rubber-to-Liquid-Öle (RTL), Biomass-to-Liquid-Öle (BTL) (vorzugsweise mit einem Gehalt an polycyclischen Aromaten von weniger als 3 Gew.-% gemäß Methode IP 346), Triglyceride (vorzugsweise Rapsöl), ein Faktis, Kohlenwasserstoffharze, und/oder Flüssig-Polymere mit einem mittleren Molekulargewicht (Bestimmung per GPC = gel permeation chromatography, in Anlehnung an BS ISO 11344:2004) zwischen 500 und 20000 g/mol. Bei der Verwendung von Mineralöl ist dieses bevorzugt ausgewählt aus der Gruppe bestehend aus DAE (Destillated Aromatic Extracts), RAE (Residual Aromatic Extract), TDAE (Treated Destillated Aromatic Extracts), MES (Mild Extracted Solvents) und naphthenischen Ölen.

Besonders bevorzugt ist in den meisten Fällen eine erfindungsgemäße Kautschukmischung, vorzugsweise wie vorstehend und/oder nachstehend als bevorzugt beschrieben, die im Wesentlichen frei ist von, vorzugsweise nicht umfassend, N-Phenyl-N'-(1,3-dimethylbutyl)-p-phenylendiamin (6PPD), N,N`-Diphenyl-p-phenylendiamin (DPPD), N-(1-phenylethyl)-N'-phenyl-p-phenylendiamin (SPPD), N,N`-Ditolyl-p-phenylendiamin (DTPD), N-(1,4-dimethylpentyl)-N'-phenyl-p-phenylendiamin (7PPD), N-Isopropyl-N'-phenyl-p-phenylendiamin (IPPD), und optional zusätzlich von 2,2,4-Trimethyl-1,2-dihydrochinolin (TMQ); weiter bevorzugt im Wesentlichen frei ist von, vorzugsweise nicht umfassend, para-Phenylendiamine und optional zusätzlich von Dihydrochinoline; am bevorzugtesten im Wesentlichen frei ist von, vorzugsweise nicht umfassend, Alterungsschutzmittel, welche keine erfindungsgemäße Verbindung ist (d.h. aus dem Stand der Technik bekannte und übliche Alterungsschutzmittel). Es ist daher besonders bevorzugt, dass die erfindungsgemäße Kautschukmischung als einziges Alterungsschutzmittel eine oder mehr als eine erfindungsgemäße Verbindung enthält, vorzugsweise wie vorstehend als bevorzugt beschrieben. Im Kontext der vorliegenden Erfindung bedeutet "im Wesentlichen frei ist von" eine Mengenobergrenze von 0,1 phr, vorzugsweise eine Gesamtmenge im Bereich von 0 phr bis 0,1 phr, besonders bevorzugt von 0,0001 phr bis 0,1 phr. Besonders bevorzugt ist die Menge Null (0), d.h., "nicht umfassend". Mit diesen sehr bevorzugt geringen Mengen, einschließlich Null (0) phr, ist es möglich, eine vergleichbare Schutzwirkung bei signifikant geringerer Toxizität zu erzielen. Hierbei ersetzt die erfindungsgemäße Verbindung die genannten im Stand der Technik bekannten und typischerweise eingesetzten para-Phenylendiamine. In einigen anderen Fällen ist eine erfindungsgemäße Kautschukmischung bevorzugt, in der zumindest teilweise Alterungsschutzmittel, die keine erfindungsgemäße Verbindung ist (besonders para-Phenylendiamine) durch eine oder mehr als eine erfindungsgemäße Verbindung ersetzt sind, vorzugsweise 50 Gew.-% oder mehr. Hierdurch wird der erfindungsgemäße Vorteil zumindest bereits teilweise erzielt, wenn auch noch nicht im optimalen Ausmaß.

Wie oben definiert ist die zusätzliche Abwesenheit von Dihydrochinolinen, vorzugsweise 2,2,4-Trimethyl-1,2-dihydrochinolin (TMQ), optional, d.h. in manchen Fällen gewünscht, in anderen nicht gewünscht. In einigen Fällen ist eine erfindungsgemäße Kautschukmischung bevorzugt, vorzugsweise wie vorstehend und/oder nachstehend als bevorzugt beschrieben, umfassend (unabhängig davon ob para-Phenylendiamine enthalten sind oder nicht) ein oder mehr als ein Dihydrochinolin, vorzugsweise 2,2,4-Trimethyl-1,2-dihydrochinolin (TMQ). Vorzugsweise ist das Dihydrochinolin, vorzugsweise das 2,2,4-Trimethyl-1,2-dihydrochinolin (TMQ), in einer Gesamtmenge im Bereich von 0,1 bis 3 phr in der erfindungsgemäßen Kautschukmischung umfasst, vorzugsweise von 0,5 bis 1,5 phr.

Ozonschutzwachse (siehe d) oben) werden separat betrachtet und sind vorzugsweise in der erfindungsgemäßen Kautschukmischung enthalten, unabhängig davon, ob Alterungsschutzmittel gemäß a) enthalten sind oder nicht.

Bei den Silan-Kupplungsagenzien (siehe c) oben) handelt es sich vorzugsweise um alle dem Fachmann bekannte Typen. Vorzugsweise umfasst die erfindungsgemäße Kautschukmischung, vorzugsweise wie vorstehend und/oder nachstehend als bevorzugt beschrieben, ein oder mehr als ein Silan-Kupplungsagenz. In manchen Fällen umfasst die erfindungsgemäße Kautschukmischung vorzugsweise ein Gemisch verschiedener Silane.

Silan-Kupplungsagenzien reagieren mit den oberflächlichen Silanolgruppen des Siliciumdioxids (insbesondere der Kieselsäure) oder anderen polaren Gruppen, entweder während des Mischens des Kautschuks bzw. der Kautschukmischung (*in situ*) oder bereits vor der Zugabe dessen zum Kautschuk während einer Vorbehandlung (Vormodifizierung).

Bevorzugte Silan-Kupplungsagenzien umfassen bifunktionale Organosilane, die am Siliciumatom mindestens eine Alkoxy-, Cycloalkoxy- oder Phenoxygruppe als Abgangsgruppe besitzen und als zusätzliche Funktionalität eine Gruppe aufweisen, die (gegebenenfalls nach Spaltung) eine chemische Reaktion mit den Doppelbindungen des Polymers eingehen kann. Bevorzugte Gruppen umfassen - SCN, -SH, -NH₂ und/oder -Sₓ- (mit x = 2 bis 8). Silane mit einer -SH Gruppe werden typischerweise auch als Mercaptosilane bezeichnet. Bevorzugte Mercaptosilane umfassen geblockte Mercaptosilane, vorzugsweise gemäß WO 99/09036 A1.

Besonders bevorzugte Silan-Kupplungsagenzien umfassen 3-Mercaptopropyltriethoxysilan, 3-Thiocyanato-propyltrimethoxysilan, 3,3`-Bis(triethoxysilylpropyl)polysulfide mit 2 bis 8 Schwefelatomen (besonders bevorzugt 3,3'-Bis(triethoxysilylpropyl)tetrasulfid (TESPT) und/oder dessen Disulfid (TESPD)) und/oder Gemische der Sulfide mit vorzugsweise jeweils unterschiedlicher Anzahl an Schwefelatomen. In einigen Fällen ist TESPT bevorzugt, besonders bevorzugt als Gemisch mit Industrieruß (Handelsname X50S^{®} der Firma Evonik).

Bevorzugte Silan-Kupplungsagenzien umfassen, zusätzlich oder alternativ, auch solche gemäß WO 2008/083241 A1, WO 2008/083242 A1, WO 2008/083243 A1 und/oder WO 2008/083244 A1. Weitere bevorzugte Silan-Kupplungsagenzien, zusätzlich oder alternativ, umfassen unter dem Namen NXT in verschiedenen Varianten von der Firma Momentive, USA, vertriebene Silane (vorzugsweise 3-Octanoylthio-1-propyltriethoxysilan) und/oder unter dem Namen VP Si 363^{®} von der Firma Evonik Industries vertriebene Silane.

Bevorzugt ist eine erfindungsgemäße Kautschukmischung, vorzugsweise wie vorstehend und/oder nachstehend als bevorzugt beschrieben, wobei der eine oder mehr als eine weitere Zusatzstoff in einer Gesamtmenge im Bereich von 3 bis 150 phr umfasst ist, bevorzugt von 4 bis 100 phr, weiter bevorzugt von 5 bis 80 phr.

Bevorzugt ist eine erfindungsgemäße Kautschukmischung, vorzugsweise wie vorstehend und/oder nachstehend als bevorzugt beschrieben, umfassend einen oder mehr als einen Aktivator (siehe b) oben); vorzugsweise eine Zinkverbindung, besonders bevorzugt Zinkoxid. Hinsichtlich der Typen an Zinkoxid gibt es vorzugsweise keine Einschränkung. Bevorzugt ist ein Granulat und/oder ein Pulver. Bevorzugt besitzt das Zinkoxid eine BET-Oberfläche bis zu 100 m²/g, vorzugsweise in einem Bereich von 1 bis 100 m²/g. In einigen Fällen ist ein Zinkoxid bevorzugt, wobei die BET Oberfläche kleiner 10 m²/g ist. In anderen Fällen ist ein Zinkoxid bevorzugt, wobei die BET Oberfläche im Bereich von 10 bis 100 m²/g liegt, besonders bevorzugt sind sogenannte "nano-Zinkoxide".

Bevorzugt ist eine erfindungsgemäße Kautschukmischung, vorzugsweise wie vorstehend und/oder nachstehend als bevorzugt beschrieben, wobei diese vulkanisiert oder teilvulkanisiert ist, vorzugsweise vulkanisiert. Dies bedeutet, dass sie vorzugsweise vulkanisiert oder mindestens teilvulkanisiert zur Anwendung kommt, besonders bevorzugt in einem Fahrzeugreifen oder in Gummiartikel, die keine Fahrzeugreifen sind (vorzugsweise technische Gummiartikel). Im Kontext der vorliegenden Erfindung werden die Begriffe "vulkanisiert" und "vernetzt" synonym verwendet. Daher ist die erfindungsgemäße Kautschukmischung vorzugsweise eine vernetzte Kautschukmischung. Dem Fachmann ist jedoch selbstverständlich klar, dass bis zur maßgeblichen Verarbeitung die Kautschukmischung typischerweise nicht vulkanisiert vorliegt.

Die Vulkanisation bzw. das Vulkanisieren der erfindungsgemäßen Kautschukmischung erfolgt bevorzugt in Anwesenheit von Schwefel und/oder Schwefelspendern, vorzugsweise unter Mitwirkung von Vulkanisationsbeschleunigern, wobei vorzugsweise einige Vulkanisationsbeschleuniger zugleich Schwefelspender sind.

Bevorzugt ist eine erfindungsgemäße Kautschukmischung, vorzugsweise wie vorstehend und/oder nachstehend als bevorzugt beschrieben, umfassend Schwefel und/oder einen oder mehr als einen Schwefelspender. Hinsichtlich der Schwefelspender gibt es keine besondere Einschränkung, so dass grundsätzlich alle für den Fachmann bekannten Schwefelspender in Betracht kommen, solange sie unter den vorliegenden Bedingungen Schwefel in entsprechender Form zur Verfügung stellen.

Bevorzugt ist eine erfindungsgemäße Kautschukmischung umfassend (alternativ oder zusätzlich zu einem Schwefelspender) einen oder mehr als einen Vulkanisationsbeschleuniger.

Bevorzugte Vulkanisationsbeschleuniger umfassen Thiazolbeschleuniger, Mercaptobeschleuniger, Sulfenamidbeschleuniger, Thiocarbamatbeschleuniger, Thiurambeschleuniger, Thiophosphatbeschleuniger, Thioharnstoffbeschleuniger, Xanthogenat-Beschleuniger und/oder Guanidin-Beschleuniger. Besonders bevorzugt ist ein oder mehr als ein Sulfenamidbeschleuniger und/oder ein oder mehr als ein Guanidin-Beschleuniger, ganz besonders ein oder mehr als ein Beschleuniger ausgewählt aus der Gruppe bestehend aus N-Cyclohexyl-2-benzothiazolsufenamid (CBS), N,N-Dicyclohexylbenzothiazol-2-sulfenamid (DCBS), Benzothiazyl-2-sulfenmorpholid (MBS), N-tert-Butyl-2-benzothiazylsulfenamid (TBBS) und Diphenylguanidin (DPG).

Bevorzugt ist eine erfindungsgemäße Kautschukmischung, vorzugsweise wie vorstehend und/oder nachstehend als bevorzugt beschrieben, umfassend einen oder mehr als einen Vulkanisationsverzögerer. Die Anwesenheit eines Vulkanisationsverzögerers trägt beispielsweise zu einem besser ausbalancierten Vulkanisationsverlauf bei.

Die erfindungsgemäße Kautschukmischung wird vorzugsweise hergestellt oder bereitgestellt, besonders bevorzugt hergestellt. Dies erfolgt vorzugsweise nach in der Kautschukindustrie üblichen Verfahren, bei denen zunächst in einer oder mehr als einer Mischstufe eine Grundmischung mit allen Bestandteilen außer (d.h. abzüglich) dem Vulkanisationssystem bereitgestellt wird, wobei das Vulkanisationssystem den Schwefel und die vulkanisationsbeeinflussenden Verbindungen umfasst, d.h. vorzugsweise die Gesamtmenge aus Schwefel, Schwefelspender, Vulkanisationsbeschleuniger und Vulkanisationsverzögerer. Durch Zugabe des Vulkanisationssystems in einer letzten Mischstufe wird eine Fertigmischung erzeugt, welche vorzugsweise durch Extrusion und/oder Kalandrieren weiterverarbeitet und in die gewünschte Form gebracht wird. Die dabei erzielte Form entscheidet vorzugsweise über die anschließende Verwendung, wie z.B. in einem Fahrzeugreifen.

Bevorzugt ist eine erfindungsgemäße Kautschukmischung zur Verwendung in einem Vulkanisat.

Weiter bevorzugt ist eine erfindungsgemäße Kautschukmischung zur Verwendung in einem Fahrzeugreifen, vorzugsweise in einem äußeren oder inneren Bauteil eines Fahrzeugreifens, vorzugsweise einem äußeren Bauteil.

Bevorzugt ist eine erfindungsgemäße Kautschukmischung, vorzugsweise wie vorstehend und/oder nachstehend als bevorzugt beschrieben, umfassend einen oder mehr als einen Festigkeitsträger, vorzugsweise umfassend Fasern, Gewebe und/oder Corde.

### Fahrzeugreifen umfassend die erfindungsgemäße Kautschukmischung:

Die vorliegende Erfindung betrifft zudem einen Fahrzeugreifen, vorzugsweise einen Fahrzeugluftreifen, der die erfindungsgemäße Kautschukmischung wie vorstehend beschrieben, vorzugsweise wie vorstehend als bevorzugt beschrieben, in wenigstens einem Bauteil aufweist, bevorzugt in wenigstens einem äußeren Bauteil, wobei das äußere Bauteil bevorzugt ein Laufstreifen, eine Seitenwand und/oder ein Hornprofil ist.

Das vorstehend Gesagte zu der erfindungsgemäßen Verbindung und die erfindungsgemäße Kautschukmischung, jeweils inklusive bevorzugter Ausgestaltungen, gilt vorzugsweise *mutatis mutandis* auch für den erfindungsgemäßen Fahrzeugreifen.

Vorzugsweise ist der erfindungsgemäße Fahrzeugreifen vulkanisiert, d.h. kein Fahrzeugreifenrohling. Vorzugsweise weist der vulkanisierte Fahrzeugreifen wenigstens in einem Bauteil ein Vulkanisat wenigstens einer erfindungsgemäßen Kautschukmischung auf. Dem Fachmann ist bekannt, dass die meisten Verbindungen, wie z. B. die enthaltenen Kautschuke entweder bereits nach dem Mischen oder erst nach der Vulkanisation in chemisch veränderter Form vorliegen oder überhaupt vorliegen können.

Der erfindungsgemäße Fahrzeugreifen, vorzugsweise wie vorstehend und/oder nachstehend als bevorzugt beschrieben, umfasst vorzugsweise einen Fahrzeugluftreifen und/oder einen Vollgummireifen.

Vorzugsweise ist der erfindungsgemäße Fahrzeugreifen, vorzugsweise wie vorstehend und/oder nachstehend als bevorzugt beschrieben, ausgewählt aus der Gruppe bestehend aus Industrie- und Baustellenfahrzeugreifen, LKW-, PKW- und Zweiradreifen.

Besonders bevorzug ist ein erfindungsgemäßer Fahrzeugreifen, vorzugsweise wie vorstehend und/oder nachstehend als bevorzugt beschrieben, der die erfindungsgemäße Kautschukmischung in zwei, drei oder mehr als drei Bauteilen aufweist.

### Verwendung der erfindungsgemäßen Verbindung und erfindungsgemäßen Kautschukmischung:

Die vorliegende Erfindung betrifft ganz allgemein eine Verwendung der erfindungsgemäßen Verbindung, vorzugsweise wie vorstehend als bevorzugt beschrieben, als Alterungsschutzmittel und/oder (vorzugsweise und) Antioxidationsmittel in einer Kautschukmischung, vorzugsweise in einer erfindungsgemäßen Kautschukmischung (vorzugsweise wie vorstehend als bevorzugt beschrieben).

Die vorliegende Erfindung betrifft zudem eine Verwendung der erfindungsgemäßen Verbindung, vorzugsweise wie vorstehend und/oder nachstehend als bevorzugt beschrieben, als Alterungsschutzmittel und/oder Antioxidationsmittel (bevorzugt als Alterungsschutzmittel), vorzugsweise in
- Fahrzeugreifen, besonders bevorzugt Fahrzeugluftreifen;
- Gummiartikeln, die keine Fahrzeugreifen sind, besonders bevorzugt ausgewählt aus der Gruppe bestehend aus Luftfedern, Bälgen, Bändern, Gurten, Riemen, Schläuchen, Profilen, Dichtungen, Membranen, taktilen Sensoren für medizinische Anwendungen, taktilen Sensoren für Roboteranwendungen, Schuhsohlen und Teilen einer Schuhsohle;
- Ölen und/oder Fetten; und/oder
- Kraft- und/oder Schmierstoffen.

Das vorstehend Gesagte zu der erfindungsgemäßen Verbindung (inklusive bevorzugter Ausgestaltungen) sowie der erfindungsgemäßen Kautschukmischung (inklusive bevorzugter Ausgestaltungen) gilt vorzugsweise *mutatis mutandis* auch für die erfindungsgemäße Verwendung als Alterungsschutzmittel und/oder Antioxidationsmittel.

Bevorzugte Bänder umfassen Förderbänder.

In einigen Fällen ist die Verwendung der erfindungsgemäßen Verbindung als Alterungsschutzmittel und/oder Antioxidationsmittel in Ölen, Fetten, Kraft- und/oder Schmierstoffen bevorzugt, besonders bevorzugt in Verbindung mit einer Verwendung für Motoren.

Die vorliegende Erfindung betrifft zudem eine Verwendung der erfindungsgemäßen Kautschukmischung, vorzugsweise wie vorstehend und/oder nachstehend als bevorzugt beschrieben, zur Herstellung eines
- Fahrzeugreifens, vorzugsweise eines Fahrzeugluftreifens; und/oder
- Gummiartikels, der kein Fahrzeugreifen ist, besonders bevorzugt ausgewählt aus der Gruppe bestehend aus Luftfedern, Bälgen, Bändern, Gurten, Riemen, Schläuchen, Profilen, Dichtungen, Membranen, taktilen Sensoren für medizinische Anwendungen, taktilen Sensoren für Roboteranwendungen, Schuhsohlen und Teilen einer Schuhsohle.

Das vorstehend Gesagte zu der erfindungsgemäßen Verbindung (inklusive bevorzugter Ausgestaltungen) sowie der erfindungsgemäßen Kautschukmischung (inklusive bevorzugter Ausgestaltungen) gilt vorzugsweise *mutatis mutandis* auch für die erfindungsgemäße Verwendung zur Herstellung wie vorstehend beschrieben. Das nachfolgend Gesagte gilt *mutatis mutandis* vorzugsweise auch für den erfindungsgemäßen Fahrzeugreifen.

Bevorzugt ist die Verwendung zur Herstellung aller Reifenbauteile (einschließlich äußerer und/oder innerer Bauteile), vorzugsweise zur Herstellung eines äußeren Bauteils, besonders bevorzugt zur Herstellung eines Hornprofils, Laufstreifens und/oder einer Seitenwand. Besonders bevorzugt ist eine Verwendung zur Herstellung einer Cap eines Laufstreifens der eine Cap/Base-Konstruktion aufweist. Eine unterschiedliche Formgebung während der Extrusion bzw. des Kalandrierens entscheidet vorzugsweise über die Art des Bauteils. Bevorzugte innere Bauteile umfassen einen Squeegee, eine Innenschicht, ein Kernprofil, einen Gürtel, eine Schulter, ein Gürtelprofil, eine Karkasse, einen Wulstverstärker, ein Wulstprofil und/oder eine Bandage. Kautschukmischungen für innere Bauteile werden typischerweise auch als Body-Mischungen bezeichnet. Die unterschiedlichen Bauteile mit ihren jeweiligen Formen und im nicht-vulkanisierten Zustand dienen typischerweise dem Aufbau eines Reifenrohlings. Der noch nichtvulkanisierte Reifenrohling wird anschließend vorzugsweise vulkanisiert.

Die erfindungsgemäße Verwendung betrifft auch die Herstellung von Gummiartikeln, die keine Fahrzeugreifen sind. Vorzugsweise weisen diese Gummiartikel einen Aufbau aus mehreren Lagen auf, besonders bevorzugt umfasst eine oder mehr als eine Lage mindestens einen Festigkeitsträger.

Bevorzugte Bänder umfassen Gummibänder und/oder Förderbänder.

### Verfahren zur Herstellung der erfindungsgemäßen Verbindung:

Die vorliegende Erfindung betrifft zudem ein Verfahren zur Herstellung einer erfindungsgemäßen Verbindung, vorzugsweise einer Verbindung gemäß Formel (I), umfassend die Verfahrensschritte:
i) Herstellen oder Bereitstellen einer Verbindung gemäß Formel (B1): 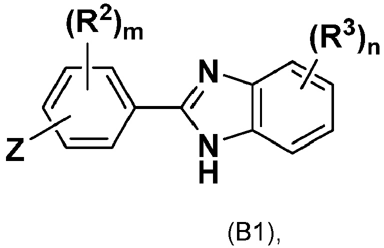
ii) Umsetzung der Verbindung gemäß Formel (B1) mit
   - Wasserstoff und einem Keton oder Aldehyd, bevorzugt einem Keton, zu der Verbindung gemäß Formel (I): 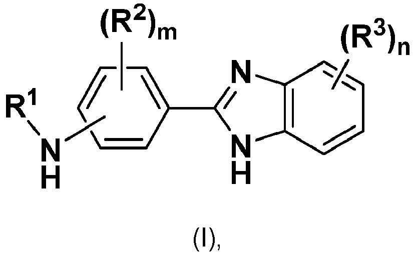
wobei
- R¹ ausgewählt ist aus der Gruppe bestehend aus
   - xi) aromatischen Resten, und
   - xii) aliphatischen C₃- bis C₁₂-Resten,
- R² und R³ gleich oder verschieden sind und unabhängig voneinander ausgewählt sind aus der Gruppe bestehend aus
   aliphatischen C₁- bis C₁₂-Resten, aromatischen Resten, Halogen-Resten, Cyano-Resten, Ester-Resten, Keton-Resten, Ether-Resten und Thioether-Resten,
- m 0 oder 1 oder 2 oder 3 oder 4 ist,
- n 0 oder 1 oder 2 oder 3 oder 4 ist, und
- Z ausgewählt ist aus der Gruppe bestehend aus Halogenen, Sulfonaten, Sulfonat-basierten Abgangsgruppen, Amino und Nitro, vorzugsweise aus Amino und Nitro.

Das vorstehend Gesagte zu der erfindungsgemäßen Verbindung (vorzugsweise und insbesondere zu erfindungsgemäßen Verbindungen, die vorstehend als bevorzugt definiert werden) gilt vorzugsweise *mutatis mutandis* auch für das erfindungsgemäße Verfahren zur Herstellung. Dies bedeutet, dass für R¹, R², R³, m und n sämtliche obigen im Zusammenhang mit der Beschreibung der erfindungsgemäßen Verbindung getroffenen Ausführungen gelten, inklusive bevorzugter Ausgestaltungen und hinsichtlich der Möglichkeiten jeglicher Abstufung bei der Bevorzugung und Kombination dieser Merkmale.

Vorzugsweise umfassen in Z die Halogene Chlor, Brom oder Iod, vorzugsweise Chlor. Besonders bevorzugt umfasst es nicht Fluor.

Vorzugsweise umfassen in Z die Sulfonat-basierten Abgangsgruppen Triflat, Nonaflat, Mesylat oder Tosylat.

Sehr bevorzugt ist Z Nitro oder Amin, vorzugsweise Amin. Dies bedeutet, dass sehr bevorzugte Ausgangsverbindungen entsprechende Amino- bzw. Nitro-Benzimidazole sind.

Bevorzugt ist ein erfindungsgemäßes Verfahren, vorzugsweise wie vorstehend und/oder nachstehend als bevorzugt beschrieben, wobei die Umsetzung in Schritt ii) mit Wasserstoff mittels eines Katalysators erfolgt, vorzugsweise mittels eines Hydrierungskatalysators. Dies bedeutet, dass in Schritt ii) ein solcher Katalysator vorzugsweise verwendet bzw. eingesetzt wird. In manchen Fällen wird ein "Hydrierungskatalysator" auch als "Hydrierkatalysator" bezeichnet, wobei im Kontext der vorliegenden Erfindung beide Begriffe den gleichen Bedeutungsumfang haben.

Bevorzugt ist ein erfindungsgemäßes Verfahren, vorzugsweise wie vorstehend und/oder nachstehend als bevorzugt beschrieben, wobei in Schritt ii) der Katalysator, vorzugsweise der Hydrierungskatalysator, ein Metall umfasst, vorzugsweise ein oder mehr als ein Metall ausgewählt aus der Gruppe bestehend aus Nickel, Kupfer, Eisen, Chrom, Aluminium, Palladium und Platin. Besonders bevorzugt umfasst der Katalysator, vorzugsweise der Hydrierungskatalysator, ein Edelmetall, besonders bevorzugt Palladium (Pd) und/oder Platin (Pt). Mit anderen Worten, besonders bevorzugt ist ein Edelmetallkatalysator.

Bevorzugt wird das Metall, vorzugsweise das Edelmetall, auf Kohle (C) eingesetzt. Besonders bevorzugte Katalysatoren umfassen Palladium auf Kohle (Pd/C), Platin auf Kohle (Pt/C), Raney-Nickel und/oder Kupferchromit, insbesondere bevorzugt ist Palladium auf Kohle (Pd/C) und/oder Platin auf Kohle (Pt/C).

Bevorzugt ist ein erfindungsgemäßes Verfahren, vorzugsweise wie vorstehend und/oder nachstehend als bevorzugt beschrieben, wobei die Umsetzung in Schritt ii) mit Wasserstoff für eine Dauer von 1 bis 30 Stunden, bevorzugt von 3 bis 22 Stunden, besonders bevorzugt von 5 bis 16 Stunden, insbesondere bevorzugt von 8 bis 13 Stunden.

Bevorzugt ist ein erfindungsgemäßes Verfahren, vorzugsweise wie vorstehend und/oder nachstehend als bevorzugt beschrieben, wobei die Umsetzung mit Wasserstoff in Schritt ii) in einem Druckreaktor erfolgt, vorzugsweise in einem Autoklav.

Besonders bevorzugt ist ein erfindungsgemäßes Verfahren, wobei die Umsetzung in Schritt ii)
- mittels eines Katalysators erfolgt; und/oder (vorzugsweise und)
- bei einer Temperatur von 40 bis 190°C erfolgt, bevorzugt von 55 bis 170°C, besonders bevorzugt von 70 bis 150°C, insbesondere bevorzugt von 85 bis 130°C; und/oder (vorzugsweise und)
- bei einem Druck von 20 bis 70 bar erfolgt, bevorzugt von 28 bis 57 bar, besonders bevorzugt von 35 bis 45 bar.

In dem erfindungsgemäßen Verfahren handelt es sich bei dem Keton in Schritt ii) um das Keton-Derivat des späteren Restes R¹; bei einem Aldehyd entsprechend um das Aldehyd-Derivat. Besonders bevorzugt ist ein Keton, am meisten bevorzugt Methylisobutylketon (MIBK).

Bevorzugt ist ein erfindungsgemäßes Verfahren, vorzugsweise wie vorstehend und/oder nachstehend als bevorzugt beschrieben, wobei in Schritt ii) ein Lösungsmittel eingesetzt wird, wobei vorzugsweise das Lösungsmittel mit dem Keton bzw. Aldehyd identisch ist oder vom Keton bzw. Aldehyd verschieden ist (vorzugsweise identisch).

Ist das Keton bzw. das Aldehyd identisch mit dem Lösungsmittel, ist das Lösungsmittel zudem Reaktionspartner. Dies ist vorzugsweise der Fall, wenn das Aldehyd bzw. das Keton unter Reaktionsbedingungen in flüssiger Form vorläge. Liegt das Keton bzw. das Aldehyd unter Reaktionsbedingungen nicht in flüssiger Form vor, ist das Lösungsmittel vorzugsweise inert, d.h. es ist selbst kein Reaktionspartner. Bevorzugte inerte Lösungsmittel umfassen Toluol, ein Dioxan (vorzugsweise 1,4-Dioxan), 2-Methyltetrahydrofuran (2-MTHF) und/oder Xylol. Dies ist vorzugsweise der Fall, wenn das Aldehyd bzw. das Keton unter Reaktionsbedingungen in fester Form vorläge. In letzterem Fall wird das Keton bzw. Aldehyd vorzugsweise nur in stöchiometrisch erforderlichen Mengen als Reaktant eingesetzt (in manchen Fällen vorzugsweise in leichtem Überschuss).

Besonders bevorzugt ist ein erfindungsgemäßes Verfahren, vorzugsweise wie vorstehend und/oder nachstehend als bevorzugt beschrieben, wobei das Keton bzw. das Aldehyd, besonders bevorzugt das Keton, in Schritt ii) in flüssiger Form vorliegt, besonders bevorzugt als Lösungsmittel und Reaktionspartner. Hierdurch kann auf eine zusätzliche Verbindung, wie Toluol oder Xylol, verzichtet werden.

Bevorzugt ist ein erfindungsgemäßes Verfahren, vorzugsweise wie vorstehend und/oder nachstehend als bevorzugt beschrieben, wobei nach Schritt ii) eine oder mehr als eine Aufreinigung erfolgt, vorzugsweise eine Filtration, eine Chromatographie, ein Umkristallisieren und/oder ein Waschen mit einem Lösungsmittel. Ein bevorzugtes Lösungsmittel, welches vorzugsweise verschieden ist von dem Lösungsmittel, welches in Schritt ii) eingesetzt wird, umfasst einen Alkohol und/oder 2-MTHF, vorzugsweise Methanol und/oder Ethanol, besonders bevorzugt Ethanol. Bevorzugt ist eine Chromatographie umfassend Kieselgel. Bevorzugt ist ein Umkristallisieren aus Kohlenwasserstoffen, vorzugsweise aus C5 bis C20 aliphatischen Verbindungen, besonders bevorzugt aus Cyclohexan und/oder Cycloheptan.

Nachfolgend wird die Erfindung durch Ausführungsbeispiele näher erläutert.

### Synthese einer Verbindung gemäß Formel (I):

Die Verbindung gemäß Formel (III), als beispielhafte Ausgestaltung einer Verbindung gemäß Formel (I), wurde auf folgende konkrete Weise in mehreren Stufen dargestellt:

### 1. Stufe (Synthese von 4-(1H-Benzo[d]imidazol-2-yl)anilin):

4-(1*H*-Benzo[d]imidazol-2-yl)anilin wurde gemäß K. V. Chikkula, E. J. Biomed. Pharm. Sci. 2017, 4(8), 749-758 dargestellt, wobei anstelle von Phosphorsäure Polyphosphorsäure und eine niedrigere Temperatur von 120°C statt 200°C angewendet wurde.

### 2. Stufe (4-(1H-benzo[d]imidazol-2-yl)-N-(1,3-Dimethylbutylamino)anilin):

Die in der 1. Stufe erhaltene Verbindung wurde in der 2. Stufe mittels eines Palladium-auf-Kohle (Pd/C) Katalysators gemäß nachfolgendem Schema umgesetzt.

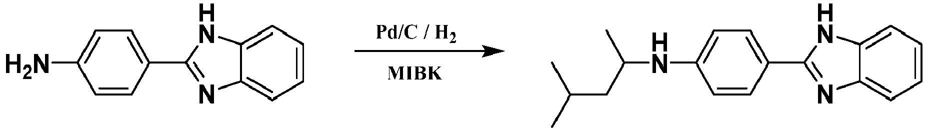

In einem mit Tefloninliner bestückten Edelstahlautoklaven wurden 0.52 g (2.47 mmol, 0.8 Äq) 4-(1*H*-Benzo[d]imidazol-2-yl)anilin, 0.21 g Palladium auf Kohle (5%; 0.4 g auf 4.67 mmol Substrat) und 20.0 mL Methylisobutylketon eingewogen. Anschließend wurden 40 bar Wasserstoff angelegt und das Reaktionsgemisch bei 120°C für 10 Stunden gerührt. Nach Beendigung der Reaktion wurde der überschüssige Wasserstoff abgelassen. Als Reaktionsprodukt wurde eine Suspension erhalten, die über Celite filtriert und anschließend mit Ethanol gewaschen wurde. Das daraus resultierende Filtrat wurde bis zur Trockne eingeengt und anschließend im Vakuum weiter getrocknet. Das resultierende, getrocknete Produkt wurde mittels Säulenchromatographie aufgereinigt (Dichlormethan/Methanol; 100:0 => 95:5). Als gereinigtes Produkt wurde ein weißer bis hellbrauner Feststoff erhalten; Ausbeute 0.25 g (34 % d. Th.).

¹H-NMR (500 MHz, DMSO-*d6*) δ = 12.41 (s, 1H), 7.93 - 7.85 (m, 2H), 7.54 (d, *J* = 6.9 Hz, 1H), 7.46 - 7.40 (m, 1H), 7.16 - 7.06 (m, 2H), 6.71 - 6.64 (m, 2H), 5.89 (d, *J* = 8.4 Hz, 1H), 3.56 (dqt, *J* = 12.6, 8.2, 4.4 Hz, 1H), 1.78 - 1.68 (m, *J* = 6.7 Hz, 1H), 1.48 (dt, *J* = 14.0, 7.1 Hz, 1H), 1.26 (ddd, *J* = 13.6, 7.4, 6.3 Hz, 1H), 1.12 (d, *J* = 6.2 Hz, 3H), 0.93 (d, *J* = 6.6 Hz, 3H), 0.88 (d, *J* = 6.6 Hz, 3H).

¹³C-NMR (126 MHz, DMSO-*d6*) δ = 153.0, 150.3, 144.6, 135.4, 128.3, 121.7, 121.5, 118.3, 117.1, 112.2, 111.0, 46.3, 45.7, 25.0, 23.1, 23.1, 21.2.
Ergebnis Massenspektrometrie: ESI-MS [M+H]⁺ = 294

Die Verbindungen der Formel (I) im Allgemeinen bzw. der Formel (III) im Besonderen beruhen auf einer Phenyl-Benzimidazol-Grundstruktur. Im Vergleich dazu basiert 6PPD auf einer Diphenylamin-Grundstruktur. Phenyl-Benzimidazol ist deutlich weniger umwelt- und gesundheitsschädlich als 6PPD bzw. weitere Vertreter dieser bekannten Verbindungsklasse.

Die erfindungsgemäße Verbindung gemäß Formel (III) als Vertreter der erfindungsgemäßen Verbindung gemäß Formel (I) weist eine ausreichende Löslichkeit in Kautschukmischungen auf.

Für die (hier besonders bevorzugte) Anwendung in einer Kautschukmischung für Fahrzeugreifen wird die erfindungsgemäße Verbindung gemäß Formel (I), repräsentiert durch eine Verbindung gemäß Formel (III), beispielsweise anstelle der im Stand der Technik bekannten Alterungsschutzmittel, wie 6PPD, 7PPD oder IPPD usw., auf eine für den Fachmann bekannte Weise in einer der Mischstufen bei der Herstellung der Kautschukmischung zugegeben.

Die Verbindung gemäß Formel (III) wird hierzu beispielsweise in verschiedenen Mengen (siehe Tabelle 2) eingemischt. Die sich ergebenden erfindungsgemäßen Beispiele sind mit E1 und E2 gekennzeichnet.

Als Vergleich dienen Kautschukmischungen enthaltend 6PPD anstelle der Verbindung gemäß Formel (III) als Alterungsschutzmittel bei sonst gleicher Zusammensetzung, wobei zwischen V1 und E1 sowie V2 und E2 jeweils ein molgleicher Austausch vorgenommen wurde. Die Mengen in Tabelle 2 sind in der Einheit phr angegeben. Die Referenz (Ref.) ist ohne Alterungsschutzmittel.

Bei sämtlichen Mischungen beträgt die Summe der Mengen an Alterungsschutzmittel (6PPD bzw. Verbindung gemäß Formel (III)) und Weichmacheröl MES 10 phr.

**Tabelle 2, beispielhafte Kautschukmischungen [phr]**

| **Bestandteil** | **V1** | **E1** | **V2** | **E2** | **Ref.** |
|---|---|---|---|---|---|
| NR | 100 | 100 | 100 | 100 | 100 |
| Ruß N 339 | 50 | 50 | 50 | 50 | 50 |
| MES | 8,0 | 7,7 | 5,0 | 4,2 | 10 |
| 6PPD | 2,0 | - | 5,0 | - | - |
| Verbindung Formel (III) | - | 2,3 | - | 5,8 | - |
| ZnO | 3 | 3 | 3 | 3 | 3 |
| Stearinsäure | 2 | 2 | 2 | 2 | 2 |
| TBBS | 1,2 | 1,2 | 1,2 | 1,2 | 1,2 |
| Schwefel | 1,2 | 1,2 | 1,2 | 1,2 | 1,2 |

Die Beispiele erfindungsgemäßer Kautschukmischungen zeigen einen Alterungsschutz (Daten nicht gezeigt). Die Referenz hingegen zeigt eine insgesamt ungenügende Alterungsschutzwirkung.

## Patentansprüche

1. Verbindung gemäß Formel (I): 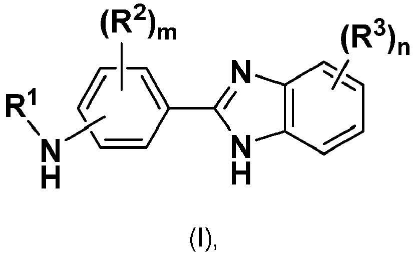 wobei
- R¹ ausgewählt ist aus der Gruppe bestehend aus
- xi) aromatischen Resten, und
- xii) aliphatischen C₃- bis C₁₂-Resten,
- R² und R³ gleich oder verschieden sind und unabhängig voneinander ausgewählt sind aus der Gruppe bestehend aus
aliphatischen C₁- bis C₁₂-Resten, aromatischen Resten, Halogen-Resten, Cyano-Resten, Ester-Resten, Keton-Resten, Ether-Resten und Thioether-Resten,
- m 0 oder 1 oder 2 oder 3 oder 4 ist, und
- n 0 oder 1 oder 2 oder 3 oder 4 ist.

2. Verbindung nach Anspruch 1, wobei sie eine Struktur gemäß Formel (II) aufweist 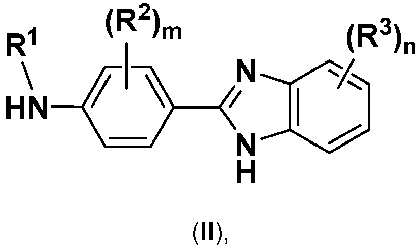 wobei R¹, R², R³, m und n wie in Anspruch 1 definiert sind.

3. Verbindung nach Anspruch 1 oder 2, wobei n und m unterschiedlich oder identisch sind.

4. Verbindung nach einem der Ansprüche 1 bis 3, wobei m und n gleich Null sind.

5. Verbindung nach einem der Ansprüche 1 bis 3, wobei m gleich Null ist und n gleich 1 ist, und vorzugsweise R³ ein aliphatischer C₁- bis C₁₂-Rest ist.

6. Verbindung nach einem der Ansprüche 1 bis 5, wobei R¹ ein tertiäres Kohlenstoffatom umfasst mit dem es an das Stickstoffatom (N) gebunden ist.

7. Verbindung nach einem der Ansprüche 1 bis 6, wobei R¹ ein verzweigter oder cyclischer Alkyl-Rest ist.

8. Verbindung nach einem der Ansprüche 1 bis 7, wobei R¹ 1,3-Dimethylbutyl, Phenyl, Benzyl oder Cyclohexyl ist, vorzugsweise 1,3-Dimethylbutyl.

9. Verbindung nach einem der Ansprüche 1 bis 8, wobei sie eine Struktur gemäß Formel (III) aufweist:

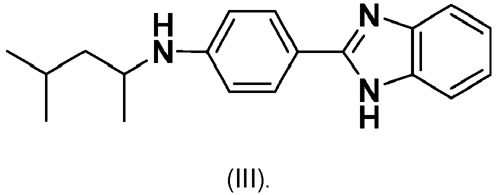

10. Kautschukmischung enthaltend die Verbindung nach einem der Ansprüche 1 bis 9, vorzugsweise umfassend einen oder mehr als einen Dienkautschuk.

11. Fahrzeugreifen, vorzugsweise einen Fahrzeugluftreifen, der die Kautschukmischung nach Anspruch 10 in wenigstens einem Bauteil aufweist, bevorzugt in wenigstens einem äußeren Bauteil, wobei das äußere Bauteil bevorzugt ein Laufstreifen, eine Seitenwand und/oder ein Hornprofil ist.

12. Verwendung der Verbindung nach einem der Ansprüche 1 bis 9 als Alterungsschutzmittel und/oder Antioxidationsmittel, vorzugsweise in
- Fahrzeugreifen, besonders bevorzugt Fahrzeugluftreifen;
- Gummiartikeln, die keine Fahrzeugreifen sind, besonders bevorzugt ausgewählt aus der Gruppe bestehend aus Luftfedern, Bälgen, Bändern, Gurten, Riemen, Schläuchen, Profilen, Dichtungen, Membranen, taktilen Sensoren für medizinische Anwendungen, taktilen Sensoren für Roboteranwendungen, Schuhsohlen und Teilen einer Schuhsohle;
- Ölen und/oder Fetten; und/oder
- Kraft- und/oder Schmierstoffen.

13. Verwendung der Kautschukmischung nach Anspruch 10 zur Herstellung eines
- Fahrzeugreifens, vorzugsweise eines Fahrzeugluftreifens; und/oder
- Gummiartikels, der kein Fahrzeugreifen ist, besonders bevorzugt ausgewählt aus der Gruppe bestehend aus Luftfedern, Bälgen, Bändern, Gurten, Riemen, Schläuchen, Profilen, Dichtungen, Membranen, taktilen Sensoren für medizinische Anwendungen, taktilen Sensoren für Roboteranwendungen, Schuhsohlen und Teilen einer Schuhsohle.

14. Verfahren zur Herstellung einer Verbindung gemäß Formel (I), umfassend die Verfahrensschritte:
i) Herstellen oder Bereitstellen einer Verbindung gemäß Formel (B1): 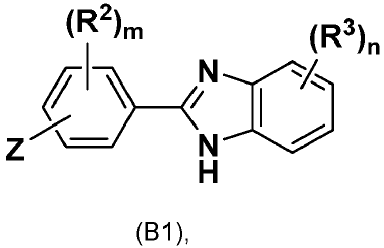
ii) Umsetzung der Verbindung gemäß Formel (B1) mit
- Wasserstoff und einem Keton oder Aldehyd, bevorzugt einem Keton, zu der Verbindung gemäß Formel (I): 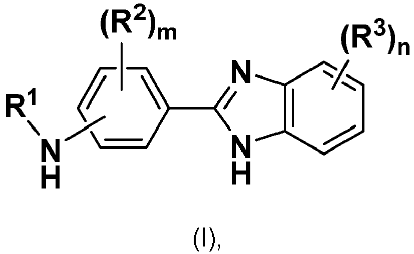
wobei
- R¹ ausgewählt ist aus der Gruppe bestehend aus
- xi) aromatischen Resten, und
- xii) aliphatischen C₃- bis C₁₂-Resten,
- R² und R³ gleich oder verschieden sind und unabhängig voneinander ausgewählt sind aus der Gruppe bestehend aus
aliphatischen C₁- bis C₁₂-Resten, aromatischen Resten, Halogen-Resten, Cyano-Resten, Ester-Resten, Keton-Resten, Ether-Resten und Thioether-Resten,
- m 0 oder 1 oder 2 oder 3 oder 4 ist,
- n 0 oder 1 oder 2 oder 3 oder 4 ist, und
- Z ausgewählt ist aus der Gruppe bestehend aus Halogenen, Sulfonaten, Sulfonat-basierten Abgangsgruppen, Amino und Nitro, vorzugsweise aus Amino und Nitro.

15. Verfahren nach Anspruch 14, wobei die Umsetzung in Schritt ii)
- mittels eines Katalysators erfolgt; und/oder
- bei einer Temperatur von 40 bis 190°C erfolgt, bevorzugt von 55 bis 170°C, besonders bevorzugt von 70 bis 150°C, insbesondere bevorzugt von 85 bis 130°C; und/oder
- bei einem Druck von 20 bis 70 bar erfolgt, bevorzugt von 28 bis 57 bar, besonders bevorzugt von 35 bis 45 bar.
